# EUROPÄISCHE PATENTSCHRIFT

(11) **EP 2 841 596 B1**
(45) Veröffentlichungstag und Bekanntmachung des Hinweises auf die Patenterteilung: **12.07.2017**
(21) Anmeldenummer: 13718341.4
(22) Anmeldetag: 26.04.2013
(51) Int. Cl.: C12Q 1/68

(54) **NACHWEISVERFAHREN FÜR MYCOBACTERIUM AVIUM SSP. PARATUBERCULOSIS**
DETECTION METHOD FOR MYCOBACTERIUM AVIUM SSP. PARATUBERCULOSIS
PROCÉDÉ DE DÉTERMINATION DE MYCOBACTERIUM AVIUM SSP. PARATUBERCULOSIS

(30) Priorität: 27.04.2012 DE 102012103730
(43) Veröffentlichungstag der Anmeldung: 04.03.2015
(73) Patentinhaber: Georg-August-Universität Göttingen Stiftung Öffentlichen Rechts, 37073 Göttingen (DE)
(72) Erfinder: CZERNY, Claus-Peter, 37136 Waake-Bösinghausen (DE); MÜNSTER, Pia, 37083 Göttingen (DE)
(74) Vertreter: Kröncke, Rolf
(86) Internationale Anmeldenummer: PCT/EP2013/058701
(87) Internationale Veröffentlichungsnummer: WO 2013/160434

(56) Entgegenhaltungen:
- EP-A1- 1 223 225
- JP-A- 2001 157 584
- Münster Pia: "Epidemiological investigations on the occurrence of Mycobacterium avium subspecies paratuberculosis in different matrices from cattle and zoo animals by IS900 polymerase chain reaction assays. Dissertation to obtain the Ph. D. degree in the Ph. D. Program for Agricultural Sciences in Goettingen (PAG", , 1. März 2012 (2012-03-01), XP055066732, Göttingen Gefunden im Internet: URL:http://ediss.uni-goettingen.de/bitstre am/handle/11858/00-1735-0000-000D-EF3D-A/m uenster.pdf?sequence=1 [gefunden am 2013-06-14]
- Pia Münster ET AL: "Detection of Mycobacterium avium ssp. paratuberculosis in ileocaecal lymph nodes collected from elderly slaughter cows using a semi-nested IS900 polymerase chain reaction", Veterinary microbiology, vol. 154, no. 1-2, 1 December 2011 (2011-12-01), pages 197-201, XP055066729, ISSN: 0378-1135, DOI: 10.1016/j.vetmic.2011.06.033
- Tim J. Bull ET AL: "A Novel Multi-Antigen Virally Vectored Vaccine against Mycobacterium avium Subspecies paratuberculosis", PLoS ONE, vol. 2, no. 11, 28 November 2007 (2007-11-28), page e1229, XP055249590, DOI: 10.1371/journal.pone.0001229

## Beschreibung

Die vorliegende Anmeldung betrifft ein Verfahren zum spezifischen Nachweis und ggf. zur Quantifizierung von *Mycobacterium avium* ssp. *paratuberculosis* (MAP) in einer Probe eines Individuums. Gemäß dem erfindungsgemäßen Verfahren wird hierzu der Nachweis des Vorhandenseins der IS900-Region in einer Probe mit Hilfe einer Nukleinsäureamplifikation und spezifischen Oligonukleotiden durchgeführt. In einem weiteren Aspekt wird vorliegend ein Test-Kit zum spezifischen Nachweis von MAP in einer Probe mittels Amplikationsverfahren bereitgestellt. Schließlich werden spezifische Oligonukleotide offenbart, die zum spezifischen Nachweis von MAP geeignet sind.

### Stand der Technik

*Mycobacterium avium* ist eine Mykobakterienart, die verschiedenste Wirte hat. *Mycobacterium avium* kann einerseits die Geflügeltuberkulose verursachen, andererseits auch bei Menschen und anderen Säugetieren als Krankheitserreger auftreten. Eine Spezies hiervon ist *Mycobacterium avium* ssp. *paratuberculosis* (MAP), ein obligat pathogenes Bakterium der Gattung *Mycobacterium.* MAP gilt aus Auslöser der Paratuberkulose (Johne'sche Krankheit), einer Erkrankung die insbesondere bei Wiederkäuern auftritt. Auch die Subspezies MAP konnte neben den Wiederkäuern und dem Menschen in weiteren Tierarten, insbesondere in Wildtieren detektiert werden, u.a. in Hasen, Vögeln, Wildkatzen, Waschbären und Ratten. Es wird weiterhin eine mögliche Beteiligung dieses Erregers bei dem Krankheitsbild "Morbus Crohn" des Menschen diskutiert.

Der Erreger MAP ist seit Anfang des 19. Jahrhunderts bekannt als Verursacher der Paratuberkulose. Bei der Paratuberkulose handelt es sich um eine chronische Darmerkrankung, die über Wochen hinweg zu einer ständigen Abmagerung führt. Im Endstadium verläuft diese Erkrankung tödlich. Überwiegend werden adulte Hauswiederkäuer, wie Rinder, Schafe und Ziegen, aber auch Wildwiederkäuer und Zootiere befallen. Üblicherweise erfolgt die Übertragung des Erregers vor allem auf neugeborene Kälber und Kälber bis zum Alter von 6 Monaten. Die Infektion findet dabei meist schleichend und unerkannt auf fäkal-oralem Wege statt und auch die Kolostralmilch erkrankter Kühe kann den Erreger enthalten. Nach einer mehrjährigen Latenzzeit mit unregelmäßiger und nicht kontrollierbarer Erregerausscheidung tritt die Krankheit typischerweise erst bei älteren Kühen auf. Derzeit gibt es keine Therapie. Impfstoffe zeigten in der Vergangenheit zweifelhafte Erfolge, so dass ein Einsatz in Deutschland zurzeit nicht erfolgt. Häufig ist der Zukauf von subklinischen bzw. persistent infizierten Tieren für die Neuinfektionen innerhalb einer Herde verantwortlich. Gerade die klinisch unauffälligen Träger sowie unerkannte Ausscheider sind die wichtigste Ursache für dauerhaft fortbestehende Bestandsinfektionen. Gerade durch die weite Verbreitung der Paratuberkulose sowie wirtschaftliche Schäden sind verbesserte Diagnostikmethoden und die Entwicklung von Bekämpfungsprogrammen aktuell gefragt. Die Paratuberkulose ist in Deutschland nach §78a Abs. 2 Tierseuchengesetz meldepflichtig.

Die Diagnostik von Paratuberkulose stellt einen wesentlichen Baustein bei der Bekämpfung und bei der Früherkennung der Paratuberkulose dar. Es gibt bereits verschiedene Testverfahren und Diagnoseverfahren, die z.B. auf Antikörper-basierten Tests beruhen. Ein Problem der kommerziell erhältlichen Tests ist allerdings die mangelnde Sensitivität und die mangelnde Spezifität. Antikörper-basierte Tests sind weiterhin ungeeignet, da serologisch negative Ausscheider aufgrund der mangelnden Sensitivität dieser Tests in den Herden verbleiben und so die Krankheit weiterhin verbreiten können.

Um zukünftig ein wirksames Eradikationsprogramm durchführen zu können, sind verlässliche Erregernachweise notwendig. Molekularbiologische Verfahren, insbesondere Verfahren auf Basis einer Nukleinsäureamplifikation sind vielfach diskutiert worden. Insbesondere molekularbiologische Verfahren auf Basis der Polymerase-Ketten-Reaktion (PCR) werden große Chancen zugerechnet. Die PCR stellt eine schnelle und zuverlässige Methode dar, um MAP-DNA in Verdachtsproben zu bestätigen. Als besonders geeignete Bereiche des MAP-Genoms wurden hierbei die Insertionssequenz IS900, aber auch Bereiche von ISMav2, hspX und F57 identifiziert. Es kristallisiert sich insbesondere das Insertionselement IS900 als ein geeigneter Bereich zur molekularen Diagnostik, insbesondere mit Hilfe von PCR-Verfahren heraus.

IS900 ist ein 1451 bp großes Fragment, das 17 Kopien innerhalb des Genoms des Referenzstammes MAP-K10 aufweist. Aufgrund dieser hohen Kopienzahl kann eine höhere Sensitivität des Nachweisverfahrens erreicht werden und macht die IS900 zu einer geeigneten Targetregion. Konventionelle und Echtzeit-PCR (real-time PCR) zum Nachweis von MAP basierend auf die Zielsequenz IS900 sind beschrieben.

So enthält die DE102007015775A1 zum spezifischen Nachweis von MAP geeignete Oligonukleotide. Weiterhin offenbart dieses Dokument entsprechende Verfahren und Test-Kits zum Nachweis von MAP. Aus der EP 2 009 118 A2 ist ebenfalls ein Verfahren zum Nachweis und zur Quantifizierung von MAP auf Basis von IS900 und F57 spezifischen Primern bekannt.

Allerdings leiden die dort beschriebenen Verfahren nach wie vor an einer mangelnden Sensitivität bei hoher Spezifität.
Bull et al., 2007, PlosOne, 11, e1229, beschreibt einen real-time PCR Assay zum Nachweis von MAP. Münster P., et al., 2011, Vet Microbiol, 154(1-2), 197-201, offenbart eine semi-rested PCR (snPCR) zum Nachweis von MAP. Eine hohe Sensitivität ist beschrieben. Allerdings erfordert eine snPCR die Durchführung von 2 PCR-Durchgängen. snPCR ist bekannt als ein Verfahren, bei dem eine hohe Kontaminationsgefahr besteht und daher für den Routineeinsatz mit einem hohen Durchsatz an Proben nicht geeignet ist.

Erforderlich ist aber, infizierte Individuen mit hoher Sensitivität und großer Spezifität nachzuweisen, um ein entsprechendes Eradikationsprogramm durchzuführen. Dieses Verfahren soll insbesondere auch in biologischen Proben, wie Kot, Organ, Milch und Gewebeproben durchführbar sein ohne vorherige umfangreiche Anzucht, welche bis zu 16 Wochen benötigt, oder Aufbereitungsschritte durchführen zu müssen.

Außerdem soll dieses Verfahren geeignet sein, eine entsprechende Automatisierung zu erlauben. Die im Stand der Technik beschrieben Verfahren erlauben dieses nicht.

### Beschreibung der Erfindung

In einem ersten Aspekt richtet sich die vorliegende Anmeldung auf ein Verfahren zum spezifischen Nachweis ggf. zur Quantifizierung von *Mycobacterium avium* ssp. *paratuberculosis* (MAP) in einer Probe eines Individuums. Das erfindungsgemäße Verfahren umfasst dabei den Schritt des Nachweises der IS900-Region eines MAP-Genoms in einer Probe mit Hilfe einer Nukleinsäureamplifikation, wobei diese Amplifikation mit den spezifischen Oligonukleotiden bestehend aus jeweils mindestens 15 aufeinanderfolgende Nukleotiden der Sequenzen gemäß Seq. ID Nr. 1 und Seq. ID Nr. 2 durchgeführt werden und ein Nachweis der IS900-Region mindestens eines MAP-Genoms das Vorhandensein von MAP in der Probe aufzeigen.

Es wurde vorliegend herausgefunden, dass die spezifischen Oligonukleotide, wie sie hier eingesetzt werden, nämlich die Oligonukleotide, die mindestens 15 aufeinanderfolgende Nukleotiden der Sequenzen gemäß Seq. ID Nr. 1 und Seq. ID Nr. 2 aufweisen, eine hervorragende Spezifität und Sensitivität gegenüber MAP in diagnostischen Verfahren auf Basis einer Nukleinsäureamplifikation erlauben.

Es ist dabei bevorzugt, dass die Oligonukleotide gemäß Seq. ID Nr. 1 und/oder Seq. ID Nr. 2 unabhängig voneinander mindestens 16, wie 17, 18 aufeinanderfolgende und insbesondere mindestens 19 Nukleotide dieser beiden Sequenzen aufweisen. Bevorzugter ist, dass zumindest eines der spezifischen Oligonukleotide gemäß Seq. ID Nr. 1 und/oder Seq. ID Nr. 2 mindestens 11, 12, 13, 14, 15, insbesondere mindestens 16, 17, 18, 19 oder sämtliche Nukleotide der genannten Sequenzen aufweist. Es ist klar, dass auch Ausführungsformen enthalten sind, bei denen ein Oligonukleotid z.B. mindestens 18 Nukleotide der Sequenz gemäß Seq. ID Nr. 1 und mindestens 19 Nukleotide der Sequenz gemäß Seq. ID Nr. 2 aufweist. Im Umfang dieser Erfindung ist jede Variation enthalten.

Das Verfahren zur Nukleinsäureamplifikation ist bevorzugt eine Polymerase-Ketten-Reaktion (PCR). Bei dieser kann es sich insbesondere um eine Echtzeit-PCR (real-time PCR)- handeln. Die PCR, z.B. in Form einer Echtzeit-PCR ist insbesondere eine quantitative PCR.

Im Gegensatz zu bekannten Verfahren, die ebenfalls auf Basis einer PCR beruhen, ist es vorliegend mit den erfindungsgemäßen Oligonukleotiden als Primerpaar möglich, in einer Amplifikationsrunde aussagekräftige Ergebnisse zu erhalten. Bisher bekannte Verfahren beruhen häufig auf den sogenannten "nested"- oder "semi-nested-PCR"-Verfahren. Hierbei erfolgt die Amplifikation in zwei Schritten, d.h. das Verfahren dauert länger und ist teurer als ein Einschrittverfahren. Weitere Vorteile der Echtzeit-PCR sind die Möglichkeit einer Quantifizierung der MAP-DNA, eine schnelle Durchführung mit geringer Kontaminationsgefahr sowie die Gewährleistung einer hohen Sensitivität und Spezifität.

Es ist insbesondere bevorzugt, dass bei der Nukleinsäureamplifikation, wie der PCR insbesondere der Echtzeit-PCR und besonders bevorzugt bei der quantitativen PCR, der Nachweis der IS900-Region mit Hilfe einer Nukleinsäuresonde erfolgt. Diese Nukleinsäuresonde ist ein üblicherweise mit einem Label oder Marker markiertes Oligonukleotid, die an die IS900-Region des MAP-Genoms und/oder an einer Nukleinsäure komplementär zu der IS900-Region des MAP-Genoms, hybridisiert.

Unter Hybridisierung wird vorliegend verstanden, dass ein Nukleinsäuremolekül, wie die Nukleinsäuresonde, sich an mindestens einem anderen Nukleinsäuremolekül, hier einem amplifizierten Genabschnitt, anlagert, wobei im Anlagerungsbereich die beiden einzelnen Stränge der Nukleinsäuren im Wesentlich vollständig komplementär sind. Es können sich auch triple Helices zwischen der Nukleinsäuresonde und vorliegender doppelsträngiger DNA ausbilden. Dem Fachmann sind bekannte Hybridisierungsverfahren bekannt. Insbesondere sind die hybridisierenden Bereiche wenigstens zu 70 % komplementär, wie mindestens 75 %, insbesondere mindestens 80 %, bevorzugt mindestens 90 %, wie mindestens 95 %, insbesondere mindestens 99 %, wie vollständig komplementär.

In einer bevorzugten Ausführungsform ist die Nukleinsäure ein Oligonukleotide mit mindestens 15 aufeinanderfolgende Nukleotiden gemäß Seq. ID Nr. 3. Es ist bevorzugt, dass die Sonde eine ist mit mindestens 16, 17, 18 aufeinanderfolgenden Nukleotiden gemäß Seq. ID Nr. 3, bevorzugt 19 Nukleotide, wie die Sequenz Seq. ID Nr. 3 selbst mit 20 Nukleotiden.

Die Sonde ist dabei in üblicher Art und Weise mit einem Label oder Marker markiert, die eine einfache Detektion mit bekannten Verfahren erlaubt. Dem Fachmann sind übliche Label oder Marker bekannt, wie Fluoreszenzfarbstoffe, z.B. FAM (5 oder 6 Carboxyfluorescein), VIC, NID, Fluorescein, Fluoresceinisothiocyanat (FITC), 6-carboxy-2', 4', 7', 4, 7-Hexachlorofluorescein (HEX), Cyaninfarbstoffe, wie Cy3, Cy5 usw., Rhodamin-Farbstoffe, Phenanthridenfarbstoffe und weitere dem Fachmann wohlbekannte Farbstoffe. Besonders geeignet für die Sonden-Detektion sind FAM und der Black-Hole-Quencher-1 (BHQ1).

Dem Fachmann sind für das jeweilige Protokoll der Nukleinsäureamplifikation, wie der PCR, der Echtzeit-PCR und insbesondere für quantitative PCR, die geeigneten Farbstoffe in Verbindung mit den geeigneten Amplifikationssystemen bekannt.

Bei der Echtzeit-PCR werden alternativ inaktive, z.B. gequenchte, Fluoreszenzfarbstoffe beigemischt, die durch die DNA-Produktion aktiviert werden. Übliche Farbstoffe, die mit der synthetisierten doppelsträngigen DNA interkalieren können, sind z.B. Ethidiumbromid oder SYBR Green. Bei der Nutzung des TaqMan Systems zur Nukleinsäureamplifikation sind die Nukleinsäuresonden solche, die durch Hydrolyse nachweisbar sind, zum Beispiel durch Bestimmung der entsprechenden Fluoreszenz. Dem Fachmann sind weitere geeignete Systeme auf Basis und z.B. LightCycler-Sonden, FRET-Sonden, Molecular Beacons oder Skorpion Primer bekannt. Das Nukleinsäureamplifikationverfahren ist insbesondere bevorzugt eine quantitative Echtzeit-PCR mit Primern gemäß Seq. ID Nr. 1 und Seq. ID Nr. 2 und einer Nukleinsäuresonde gemäß Seq. ID Nr. 3.

Bei dem erfindungsgemäßen Verfahren und auch in den erfindungsgemäßen Test-Kits können weiterhin entsprechende Kontrollen vorliegen. Diese Kontrollen beinhalten Kontrollen für die Amplifikation und/oder Kontrollen für die Aufreinigung. Diese Kontrollen können z.B. in der Probe selbst vorliegen oder in einen parallelen Ansatz durchgeführt werden. So kann beispielhaft die Sonde bei der Echtzeit-PCR mit einem gequenchten Farbstoff, wie FAM, ausgestattet sein. Gleichzeitig ist die Sonde mit einem zweiten Marker, der in einem anderen Bereich, z.B. in einem anderem Bereich Licht emittiert, nachgewiesen werden kann als der gequenchte Farbstoff und der nicht gequencht ist, ausgestattet. Dadurch ist eine Kontrolle der PCR möglich. Weiterhin kann in der Probe oder parallel dazu eine Positivkontrolle amplifiziert werden, wie ß-Aktin. Der Nachweis hiervon erfolgt mit einem Marker, der zu den ersten und zweiten Marker unterschiedlich ist, z.B. ein Cy5 Farbstoff. Dadurch ist eine Kontrolle der Reaktion möglich und erlaubt eine Verbesserung der Aussagekraft des Verfahrens. Zur Amplifikationskontrolle, welche den inhibitorischen Effekt einer klinischen Probe ausschließt, kann eine zweite Fluoreszenzfarbe (z.B. HEX, gemessen bei 533-580 nm) für die Sonde integriert werden.

Zur Quantifizierung können in Parallelansätzen weiterhin entsprechende Positivkontrollen für MAP in vorbestimmten Konzentrationen eingesetzt werden. Dem Fachmann sind die entsprechenden Versuchsansätze bekannt.

Die Nukleinsäureamplifikation erfolgt bevorzugt aus einer Probe eines Individuums. Die der Nukleinsäureamplifikation unterworfene Probe kann dabei eine Probe sein, die aus einer Vielzahl von gepoolten Proben besteht, um so ein effektives Testen auf MAP zu ermöglichen. Üblicherweise kann eine gepoolte Probe eine Mischung aus z.B. 10 bis 20 einzelnen Proben unterschiedlicher Individuen sein. Alternativ handelt es sich bei der Probe um die Probe eines einzelnen Individuums.

Unter dem Ausdruck Individuum, wird vorliegend insbesondere verstanden, ein Individuum ausgewählt aus Wiederkäuern, insbesondere Rinder, Schafe und Ziegen aber auch wilde Wiederkäuer, wie Zebra usw. Hierbei kann es sich z.B. um Zootiere handeln. Unter dem Ausdruck Individuum fallen weiterhin Menschen. So kann das Verfahren z.B. eingesetzt werden, um bei einem Menschen eine Infektion mit MAP zu testen, z.B. im Zusammenhang mit der Diagnose von Morbus Crohn.

Bei der Probe selbst kann es sich um eine ausgewählte Kot-, Milch-, Blut-, Sperma-, Gewebe- oder Organ-Probe handeln. Alternativ kann es sich auch um Umweltproben handeln, z.B. Proben auf pflanzlicher Basis oder Proben aus Bio-gasanlagen und Gewässern.

Bevorzugt handelt es sich bei der Probe um eine Kotprobe oder eine Milchprobe eines Wiederkäuers, insbesondere eines Rindes.

Die Probe kann dabei erfindungsgemäß mit geeigneten Verfahren aufgearbeitet werden. Übliche Verfahren schließen eine Aufarbeitung der Probe ein. Dem Fachmann sind geeignete Verfahren bekannt. Insbesondere kann die Aufarbeitung mit Hilfe bekannter DNA-Extraktionsverfahren erfolgen. Diese schließen die Verwendung bekannter Kits auf Basis von Tensiden und Salzen ein. Weiterhin sind mechanische DNA-Extraktionsverfahren möglich, einschließlich einer Aufarbeitung mittels Homogenisatoren etc. Insbesondere sind Verfahren geeignet, die den Einsatz von Tensiden zur Extraktion einschließen.

In einem weiteren Aspekt richtet sich die vorliegende Erfindung auf Test-Kits zum spezifischen Nachweis von MAP in einer Probe mittels Amplifikationverfahren, insbesondere mittels PCR. Diese Test-Kits umfassen Oligonukleotide zur Amplifizierung der MAP IS900-Region mit jeweils mindestens 15 aufeinanderfolgende Nukleotiden gemäß Seq. ID Nr. 1 und 2. Bevorzugt ist dieses Test-Kit eines für die PCR-Nukleinsäureamplifikation insbesondere eines für die Echtzeit-PCR. In einer besonders bevorzugten Ausführungsform handelt es sich bei dem Test-Kit um eines geeignet für die quantitative PCR, wie die quantitative Echtzeit-PCR. In einer Ausführungsform umfasst das Test-Kit weiterhin ein Oligonukleotid mit mindestens 15 aufeinanderfolgenden Nukleotiden der Nukleinsäuresonde gemäß Seq. ID Nr. 3. Dieses Test-Kit ist insbesondere geeignet zur quantitativen Analyse auf Basis von z.B. der TaqMan PCR. Das erfindungsgemäße Test-Kit kann weiterhin weitere Bestandteile zur Durchführung der Nukleinsäureamplifikation, insbesondere die notwendigen Enzyme, Nukleotide und Puffer aufweisen. Weiterhin bevorzugt kann eine Positivkontrolle mit beigefügt sein und/oder Anweisungen zur Durchführung des erfindungsgemäßen Verfahrens.

Schließlich kann das Test-Kit entsprechende Reagenzien und Mittel enthalten, die zur Gewinnung der genomischen DNA aus Proben insbesondere Kot-, Milch-, Blut-, Sperma-, Organ-, Gewebe- und Umweltproben geeignet sind, und/oder Mittel zur Durchführung der PCR, insbesondere Echtzeit-PCR.

Dem Fachmann sind geeignete Reagenzien und Mittel bekannt.

Die Anmeldung richtet sich schließlich auf Oligonukleotide zum spezifischen Nachweis und, optional, zur Quantifizierung von MAP in einer Probe eines Individuums, wobei mindestens 15 aufeinanderfolgende Nukleotide der Sequenz Seq. ID Nr. 1 und mindestens 15 aufeinanderfolgende Nukleotide der Sequenz ID Nr. 2 vorhanden sind und, ggf., mindestens 15 aufeinanderfolgende Nukleotide der Seq. ID Nr. 3.

Diese Oligonukleotide sind insbesondere in der Verwendung zum spezifischen Nachweis und optional zur Quantifizierung von MAP insbesondere mittels quantitativer Echtzeit-PCR geeignet. Die erfindungsgemäßen Oligonukleotide sind solche, wie oben ausgeführt.

Die Erfindung wird im Folgenden mit Hilfe von Beispielen näher erläutert, ohne auf sie beschränkt zu sein.

### Ausführungsbeispiel:

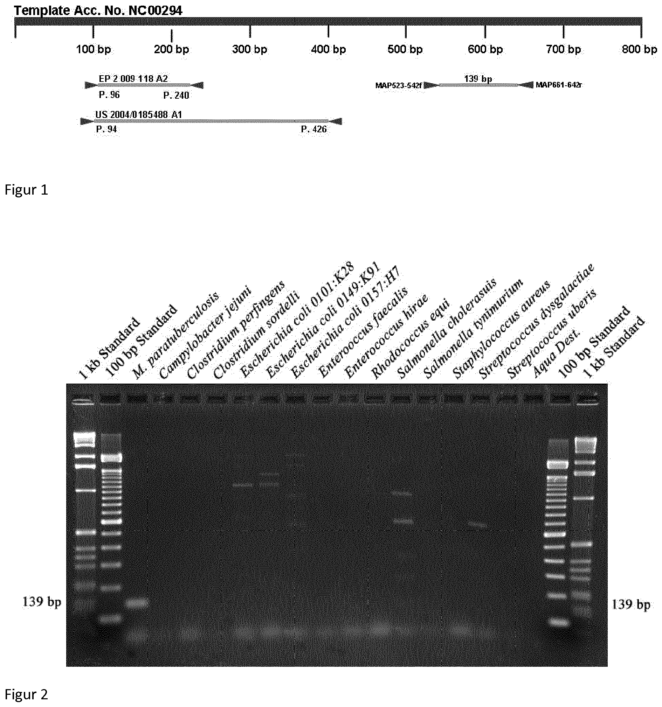

Für die Überprüfung der Spezifität der erfindungsgemäßen Primer (MAP_{523-542f}/MAP₆₆₁₋₆₄₂ᵣ₎ und die TaqMan Sonde (MAP₆₁₇₋₆₃₆ₚ) gemäß Tabelle 1 wurden 14 Mykobakterien Arten sowie 14 nicht-Mykobakterien Arten, die häufig in der Landwirtschaft und der Umwelt vorkommen, sowohl in der klassischen als auch in der Echtzeit-PCR getestet und die Spezifität bestätigt (Tabelle 2). Figur 1 zeigt die Position der erfindungsgemäßen Primer zu den Primern aus dem Stand der Technik.

**Tabelle 1: Nukleinsäuresequenzen der Primer und TaqMan Sonde zur Detektion von MAP-DNA in der Echtzeit-PCR.**

| **Primer/Sonde** | **Sequenz (5'-3')** | **Lokalisation**/**Seq**.**ID**.**No**.* |
|---|---|---|
| MAP_{523-542f} | TACCGCGGCGAAGGCAAGAC | 523-542/1 |
| MAP₆₆₁₋₆₄₂ᵣ | CGGAACGTCGGCTGGTCAGG | 661-642/2 |
| MAP₆₁₇₋₆₃₆ₚ | ATGACATCGCAGTCGAGCTG | 617-636/3 |

| | | |
|---|---|---|
| * Nach der publizierten IS900 Sequenz MAP K-10 (GenBank: AF416985), Seq. ID No. 4. | | |

**Tabelle 2: Verwendete Referenzstämme zum Spezifitäts-Nachweis der TaqMan Echtzeit-PCR.**

| **Bakterien-Spezies** | **Name/Quelle** |
|---|---|
| Mykobakterien-Arten | |
| *M. avium* ssp. *paratuberculosis* | ATCC 19698 |
| *M. avium* ssp. *avium* | ATCC 15769 |
| *M. avium* ssp. *avium* | ATCC 19421 |
| *M. avium* ssp. *avium* | ATCC 25291 |
| *M. avium* ssp. *silvaticum* | ATCC 49884 |
| *M. bovis* | ATCC 27289 |
| *M. fortuitum* | ATCC 6841 |
| *M. gordonae* | ATCC 14470 |
| *M. intracellulare* | ATCC 13950 |
| *M. kansasü* | ATCC 12478 |
| *M. marinum* | ATCC 927 |
| *M. phlei* | ATCC 354 |
| *M. scrofulaceum* | ATCC 19981 |
| *M. smegmatis* | ATCC 19420 |
| | |
| Andere Bakterien-Arten | |
| *Campylobacter jejuni* | ATCC 33560 |
| *Clostridium perfringens* | Cattle |
| *Clostridium sordelli* | Cattle |
| *Escherichia coli 0101:K28* | Cattle |
| *Escherichia coli 0149:K91* | Swine |
| *Escherichia coli 0157:H7* | ATCC 700927 |
| *Enterococcus faecalis* | ATCC 19433 |
| *Enterococcus hirae* | ATCC 10541 |
| *Rhodococcus equi* | ATCC 25694 |
| *Salmonella cholerasuis* | Cattle |
| *Salmonella thyphimurium* | Cattle |
| *Staphylococcus aureus* | Cattle |
| *Streptococcus dysgalactiae* | Cattle |
| *Streptococcus uberis* | Cattle |

Die verwendete DNA der Referenzstämme wurde aus Kulturen mit Hilfe des QIAamp Blood Kit (Quiagen, Hilden, Germany) extrahiert und mittels Nano-Drop^{®} (ND-1000 Spectrophotometer, peQLab Biotechnologie GmbH, Erlangen) gemessen. Die DNA wurde auf 1 ng/µl eingestellt. Durch den Einsatz publizierter PCR-Verfahren wurde die speziesspezifische DNA der Referenzstämme bestätigt. Als Negativkontrolle diente steriles, destilliertes Wasser.

In allen Fällen waren auf dem Agarosegel die zu erwartenden PCR-Produkte der jeweiligen Genus-spezifischen PCR deutlich zu erkennen.

Zur Abklärung der Spezifität wurden die beiden Primer (MAP_{523-542f}/MAP₆₆₁₋₆₄₂ᵣ) auf Kreuzreaktivität mit anderen Bakterienspezies in der klassischen PCR getestet (Figur 2). Für den Ansatz der PCR wurden "Ready-To-Go^{™}" PCR "Beads" (Amersham Pharmacia Biotech Europe, Freiburg) verwendet. Reaktionsansatz und -bedingungen der PCR waren wie folgt:

| | |
|---|---|
| Ready-To-GoTM PCR Beads | 1 Bead |
| Aqua dest. | 21 µl |
| Primer MAP-for (10 pmol/µl) | 1 µl |
| Primer MAP-rev (10 pmol/µl) | 1 µl |
| Template | 2 µl |

Der PCR ist ein Denaturierungsschritt bei 95ºC für 4 min vorgeschaltet.

| | | |
|---|---|---|
| Denaturierung: | 95 ºC, | 30 sec |
| Annealing: | 58-70 ºC, | 30 sec |
| Elongation: | 72 ºC, | 60 sec |

Den Abschluss der PCR bildet die Verlängerung der Elongation bei 72ºC für 7 min.

Die zu erwartenden 139 bp großen PCR-Produkte waren auf dem Agarosegel deutlich und ausschließlich bei der MAP-DNA-Probe (ATCC 19698) zu erkennen. Amplifikate im 139 bp Bereich wurden bei anderen Bakterienspezies (n = 14) nicht beobachtet. Daher können die eingesetzten Primer (MAP_{523-542f}/MAP₆₆₁₋₆₄₂ᵣ) als MAP-spezifisch angesehen werden.

Zusätzlich wurde die Spezifität mittels Sequenzierung des 139 bp langen Produktes bestätigt. Dazu wurden die PCR-Produkte in den Plasmidvektor pCR^{®} 2.1-TOPO^{®} (Invitrogen, Groningen, Niederlande) kloniert, in *E. coli* Bakterien vermehrt und mit dem Wizard^{®} Plus SV Minipreps Purification System (Promega, Mannheim) aufgereinigt. Die Nukleotidsequenzen wurden mit Hilfe von MegAlign mit der Sequenz des MAP K-10 Referenzstammes (Accession No. AE16958) verglichen. Insgesamt wurden 15 Klone in beiden Richtungen sequenziert und ausgewertet. Die IS900-Sequenzen der Amplifikate stimmten mit der publizierten bovinen MAP K-10 Sequenz zu 100% überein.

Nachdem die Spezifität der Primer (MAP_{523-542f}/MAP₆₆₁₋₆₄₂ᵣ) in der klassischen PCR anhand von Referenzstämmen (n = 14) überprüft wurde, sollte die Spezifität der Sonde (MAP₆₁₇₋₆₃₆ₚ) in Kombination mit den Primern in der Echtzeit-PCR bestätigt werden. Als Negativkontrolle diente steriles, destilliertes Wasser. Reaktionsansatz sowie Reaktionsbedingungen der Echtzeit-PCR mit TaqMan Sonde auf dem LightCycler^{™} 480 (Roche Diagnostics, Mannheim) waren wie folgt:

| | |
|---|---|
| Light Cycler^{™} DNA 480 Master | 10 µl |
| Primer MAP-for (10 pmol/µl) | 0,5 µl |
| Primer MAP-rev (10 pmol/µl) | 0,5 µl |
| Sonde (10 pmol/µl) | 1 µl |
| Aqua dest. | 3 µl |
| Template | 5 µl |

| | | |
|---|---|---|
| 95 °C | 10 min | |
| 95 °C | 15 sec | |
| 60 °C | 30 sec | 45 Zyklen |
| 72 °C | 35 sec | |

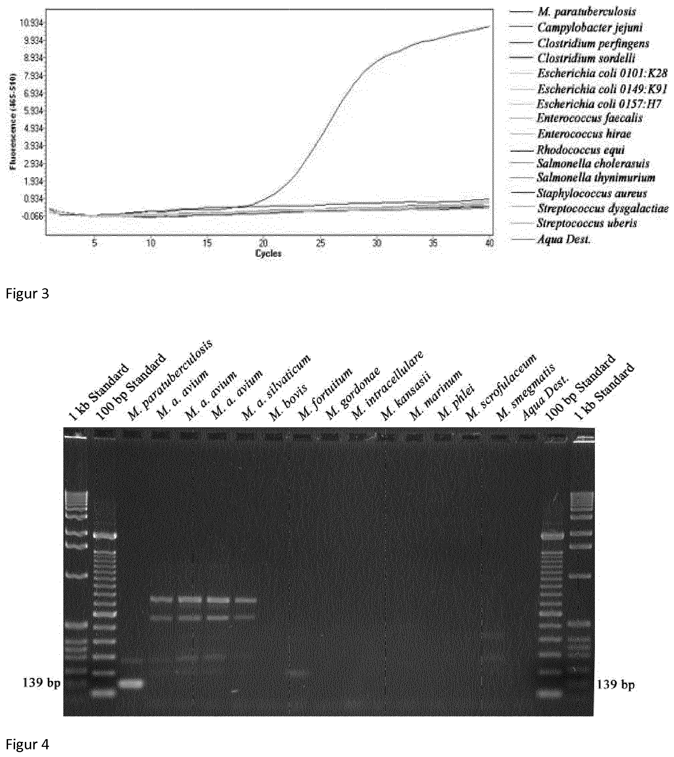

Figur 3 zeigt die Detektion von MAP mittels TaqMan Sonde (MAP₆₁₇₋₆₃₆ₚ).

Die MAP-Spezifität der Primer (MAP_{523-542f}/MAP₆₆₁₋₆₄₂ᵣ) und der TaqMan Sonde (MAP₆₁₇₋₆₃₆ₚ) konnte hinreichend belegt werden. Ein Signal war nur beim Referenzstamm MAP (ATCC 19698) sichtbar. Die Reaktion der anderen Bakterienspezies war vergleichbar mit der Negativkontrolle.

Zur weiteren Abklärung der Spezifität wurden die Primer (MAP_{523-542f}/MAP₆₆₁₋₆₄₂ᵣ) sowie die TaqMan Sonde MAP₆₁₇₋₆₃₆ₚ) auf Kreuzreaktivität mit anderen Mykobakterien-Spezies (n = 13) getestet.

Die verwendete DNA der Mykobakterien-Referenzstämme wurde aus Kulturen wie oben beschrieben aufgereinigt und mittels Nano-Drop^{®} gemessen. Die DNA wurde auf eine Konzentration von 1 ng/µl eingestellt. Durch den Einsatz eines publizierten "multiplex"-PCR-Verfahrens (Shin et al., 2010, Journal of Clinical Microbiology 48 (11), 4057-4062) wurde die speziesspezifische DNA der Referenzstämme bestätigt. Als Negativkontrolle diente steriles, destilliertes Wasser. Reaktionsansatz und Reaktionsbedingungen waren wie in der Literatur angegeben.

Die zu erwartenden PCR-Produkte der jeweiligen Mykobakterien-Arten waren auf dem Agarosegel deutlich zu erkennen. Zur weiteren Spezifitäts-Abklärung der Primer (MAP_{523-542f}/MAP₆₆₁₋₆₄₂ᵣ) wurden diese auf Kreuzreaktivität mit anderen Mykobakterien-Arten in der klassischen PCR getestet (Figur 4).

Die zu erwartenden 139 bp großen PCR-Produkte waren auf dem Agarosegel deutlich ausschließlich bei MAP-DNA (ATCC 19698) zu erkennen. Amplifikate im 139 bp Bereich traten bei anderen Mykobakterien-Spezies (n = 13) nicht auf. Daher können die eingesetzten Primer (MAP_{523-542f}/MAP₆₆₁₋₆₄₂ᵣ) gegenüber anderen Mykobakterien-Arten als MAP-spezifisch angesehen werden.

Nachdem die Spezifität der Primer (MAP_{523-542f}/MAP₆₆₁₋₆₄₂ᵣ) in der klassischen PCR anhand von Mykobakterien-Referenzstämmen (n = 13) überprüft wurde, sollte die Spezifität der Sonde (MAP₆₁₇₋₆₃₆ₚ) in Kombination mit den Primern in der Echtzeit-PCR bestätigt werden. Als Negativkontrolle diente steriles, destilliertes Wasser. Reaktionsansatz sowie Reaktionsbedingungen der Echtzeit-PCR mit TaqMan Sonde auf dem LightCycler™ 480 waren wie bereits oben definiert.

Die MAP-Spezifität der Primer (MAP_{523-542f}/MAP₆₆₁₋₆₄₂ᵣ) und der TaqMan Sonde (MAP₆₁₇₋₆₃₆ₚ) konnte auch gegenüber Mykobakterien-Spezies hinreichend belegt werden. Ein Signal war nur bei dem Referenzstamm MAP (ATCC 19698) sichtbar. Die Reaktion der anderen Mykobakterienstämme war vergleichbar mit der Negativkontrolle.

Die Nachweisgrenze bzw. Sensitivität der entwickelten TaqMan Echtzeit-PCR wurde mittels Titrationsreihen von 1 ng/µl bis 1 fg/µl Plasmid-DNA und MAP-DNA (ATCC 19698) aus Kultur ermittelt (Figur 5).

Für die Herstellung von Plasmid-DNA wurden PCR-Produkte (139 bp) wie oben beschrieben kloniert und aufgereinigt. Anschließend wurde der Nukleinsäuregehalt von Plasmid-DNA mittels NanoDrop^{®} ermittelt und Verdünnungsreihen von 1 ng/µl bis 1 fg/µl hergestellt.

Beim Einsatz von kulturell gewonnener MAP-DNA wurde eine Nachweisgrenze von 10 fg/µl erreicht. Das Genom von MAP besitzt eine Länge von 4,7 10⁶ bp (Cocito et al., 1994, Clinical Microbiology Reviews 7 (3), 328-345). Unter Verwendung der Avogadro'schen Konstante 6,022 10²³ mol⁻¹ und dem Molgewicht eines Basenpaares von 660 g × mol⁻¹ ließ sich berechnen, dass 5,15 fg einer Genomeinheit entsprechen (Münster et al., 2011, Veterinary Microbiology 154, 197-201; Münster et al., 2012). Nach dieser Berechnung lag die Nachweisgrenze mit 10 fg/µl bei ca. 2 Genomeinheiten.

Nach Überprüfung der Spezifität und Sensitivität wurde die Echtzeit-PCR auf ihre Effizienz getestet. Die Effizienz stellt ein Qualitätsmerkmal einer quantitativen Echtzeit-PCR dar. Durch Gegenüberstellung der erzielten Cp-Werte und der MAP-DNA Konzentration wurde eine Standardkurve erstellt. Eine Standardkurve gibt die logarithmische Konzentration der isolierten DNA des Erregers in den jeweiligen Verdünnungsstufen an. Die Steigung der Geraden betrug -3,42 ± 0,23, der Fehler 0,04 ± 0,02 und die Effizienz lag bei 1,97 ± 0,08 (Auswertung mittels "2nd Derivative Maximum" Methode). Damit erwies sich die lineare Standardkurve für eine quantitative Anwendung der TaqMan Echtzeit-PCR als geeignet.

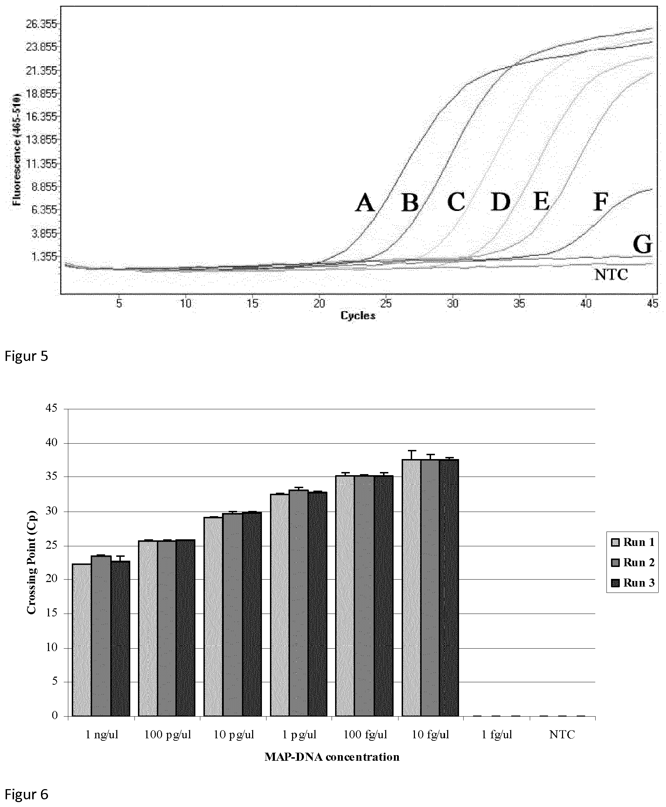

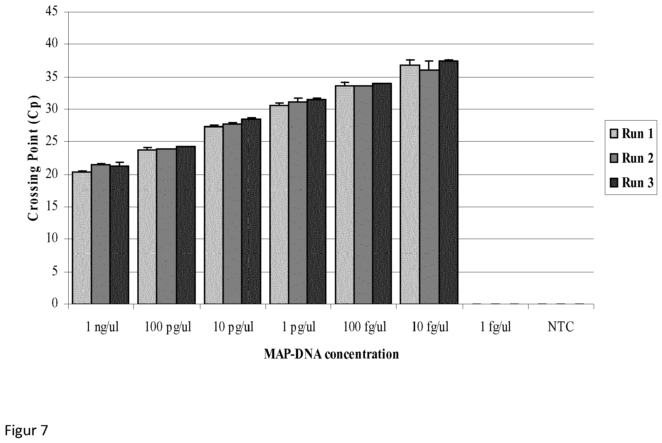

Die Reproduzierbarkeit der Echtzeit-PCR wurde anhand von 9 Wiederholungen auf dem LightCycler™ 480 ermittelt (3x3 Läufe) und mittels "2nd Derivative Maximum" sowie der "Fit Point" Methode ausgewertet. Zur Visualisierung wurden der jeweilige Mittelwert sowie die Standardabweichung der "Crossing points" (Cp), d.h. die Zyklenzahl, an denen die Fluoreszenz erstmalig signifikant über die Hintergrund-Fluoreszenz ansteigt, berechnet und als Säulendiagramm dargestellt (Figur 6 und 7).

Es konnte gezeigt werden, dass die Echtzeit-PCR reproduzierbare Ergebnisse lieferte. Alle neun Wiederholungen ergaben ähnliche Mittelwerte der Cp-Werte, wobei die Standardabweichung statistisch niedrig war (maximal ± 0,73).

Auch eine Auswertung mittels "Fit Point"-Methode erzielte reproduzierbare Ergebnisse mit gleichbleibender Sensitivität von 10 fg/µl.

Zur Prüfung der Quantifizierbarkeit der TaqMan Echtzeit-PCR bzw. Veranschaulichung der Übereinstimmung der kalkulierten mit den bekannten MAP-DNA Konzentration wurden diese berechnet und in Tabelle 3 gegenübergestellt. DNA-Konzentrationen wurden sowohl mit der "2nd Derivative Maximum"-als auch mit der "Fit Point"-Methode berechnet. Bei letztem wurden manuell die "Noise Band" auf 6,00 und der "Treshold" auf 3,00 gesetzt.

**Tabelle 3: Quantifizierung von MAP-DNA aus Kultur in log₁₀ Verdünnungsstufen mittels TaqMan Echtzeit-PCR anhand von 9 Wiederholungen (3x3 Läufe). Die Auswertung erfolgte mittels "2nd Derivative Maximum"- und "Fit Point"-Methode.**

| | **2nd Derivative Maximum** | | | **Fit Point** | |
|---|---|---|---|---|---|
| **Probe** | **Bekannte Konz.** | **Berechnete Konz.** | **Cp-Wert** | **Berechnete Konz.** | **Cp-Wert** |
| **1 ng/µl** | 10⁻⁹ | 9,17 x 10⁻¹⁰ | 22,79 ± 0,65 | 1,10 x 10⁻⁹ | 21,00 ± 0,62 |
| **100 pg**/**µl** | 10⁻¹⁰ | 1,25 x 10⁻¹⁰ | 25,76 ± 0,07 | 1,33 x 10⁻¹⁰ | 23,94 ± 0,34 |
| **10 pg**/**µl** | 10⁻¹¹ | 9,12 x 10⁻¹² | 29,61 ± 0,38 | 7,62 x 10⁻¹² | 27,88 ± 0,52 |
| **1 pg**/**µl** | 10⁻¹² | 8,19 x 10⁻¹³ | 32,82 ± 0,39 | 7,46 x 10⁻¹³ | 31,11 ± 0,54 |
| **100 fg**/**µl** | 10⁻¹³ | 1,29 x 10⁻¹³ | 35,18 ± 0,36 | 1,14 x 10⁻¹³ | 33,73 ± 0,35 |
| **10 fg**/**µl** | 10⁻¹³ | 1,05 x 10⁻¹⁴ | 37,61 ± 0,73 | 1,26 x 10⁻¹⁴ | 36,80 ± 0,94 |
| **1 fg/µl** | 10⁻¹⁵ | -- | -- | -- | -- |
| **NTC** | -- | -- | -- | -- | -- |

Ähnliche Werte der bekannten und berechneten DNA-Konzentrationen zeigten eine grundsätzliche Quantifizierbarkeit von MAP-DNA mittels TaqMan Echtzeit-PCR. Auch als die Reaktionsansätze der Verdünnungsreihe von MAP-DNA mittels "Fit Point"-Methode ("Noise Band" 6,00; "Treshold" 3,00) ausgewertet wurden, wurden bei gleich bleibender Sensitivität die Ergebnisse der berechneten DNA-Konzentrationen nicht verfälscht.

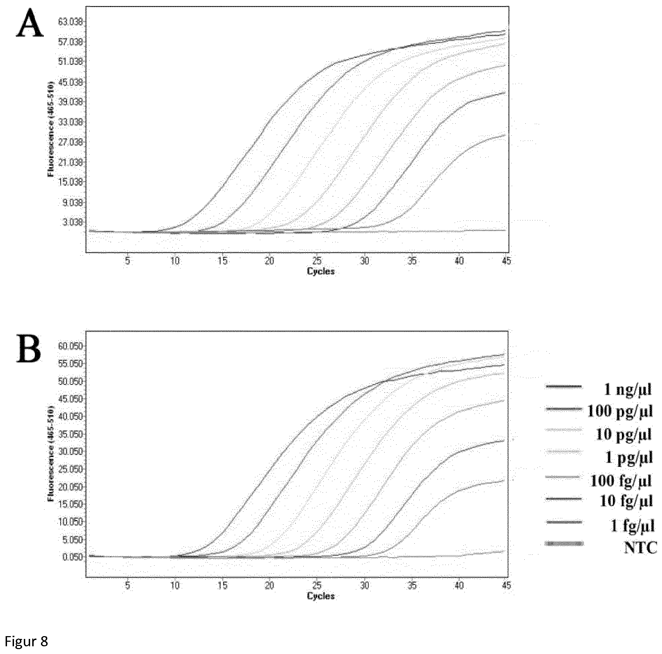

Die Stabilität bzw. Sensitivität einer PCR kann unter Feldbedingungen beeinflusst werden, wenn DNA aus Kot oder Gewebe extrahiert wird. Die mögliche inhibierende Wirkung wurde mit Hilfe von Plasmid-DNA Verdünnungsreihen (1 ng/µl bis 1 fg/µl), welche mit DNA aus negativem Kot versetzt wurden, ausgeschlossen (Figur 8).

Auch als die Reaktionsansätze der Verdünnungsreihe von Plasmid-DNA mit DNA aus MAP-negativem Kot hinterlegt wurden, wurde bei gleich bleibender Sensitivität das Ergebnis nicht verfälscht. Für die Veranschaulichung der Übereinstimmung der kalkulierten mit den bekannten MAP-DNA Konzentrationen wurde diese berechnet und in Tabelle 4 gegenübergestellt.

**Tabelle 4: Berechnete Konzentrationen und ermittelte Cp-Werte von Plasmid-DNA in log₁₀ Verdünnungsstufen (1 ng/µl bis 1 fg/µl) mittels TaqMan Echtzeit-PCR.**

| | | **H₂O** | | **Kot-DNA** | |
|---|---|---|---|---|---|
| **Probe** | **Bekannte Konz.** | **Berechnete Konz.** | **Crossing Point** | **Berechnete Konz.** | **Crossing Point** |
| **1 ng**/**µl** | 10⁻⁹ | 9,63 x 10⁻¹⁰ | 11,61 ± 0,07 | 9,97 x 10⁻¹⁰ | 13,53 ± 0,12 |
| **100 pg/µl** | 10⁻¹⁰ | 1,08 x 10⁻¹⁰ | 15,27 ± 0,22 | 1,05 x 10⁻¹⁰ | 16,88 ± 0,02 |
| **10 pg/µl** | 10⁻¹¹ | 9,67 x 10⁻¹² | 19,22 ± 0,11 | 9,42 x 10⁻¹² | 20,47 ± 0,14 |
| **1 pg/µl** | 10⁻¹² | 7,51 x 10⁻¹³ | 23,33 ± 0,24 | 9,89 x 10⁻¹³ | 23,83 ± 0,03 |
| **100 fg/µl** | 10⁻¹³ | 9,00 x 10⁻¹⁴ | 26,63 ± 0,15 | 1,03 x 10⁻¹³ | 27,20 ± 0,07 |
| **10 fg/µl** | 10⁻¹⁴ | 1,14 x 10⁻¹⁴ | 29,75 ± 0,06 | 9,83 x 10⁻¹⁵ | 30,13 ± 0,17 |
| **1 fg/µl** | 10⁻¹⁵ | 9,85 x 10⁻¹⁶ | 33,33 ± 0,07 | 1,01 x 10⁻¹⁵ | 32,34 ± 0,03 |
| **NTC** | -- | -- | -- | -- | -- |

Trotz der Hinterlegung mit DNA aus MAP-negativem Kot sind keine gravierenden Verschiebungen der Werte erkennbar. Ähnliche Werte der bekannten und berechneten DNA-Konzentrationen zeigen eine grundsätzliche Einsetzbarkeit der TaqMan Echtzeit-PCR zur Quantifizierung von MAP-DNA unabhängig von der Matrix Kot.

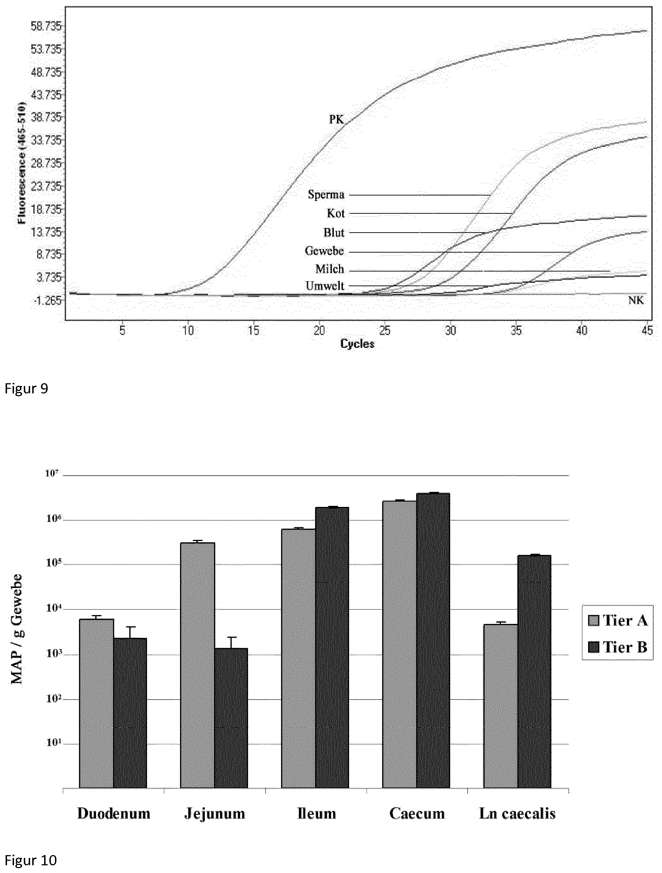

Für die vielseitige Anwendbarkeit der Echtzeit-PCR in der Routinediagnostik sowie in der Forschung muss der Nachweis von MAP-DNA in unterschiedlichen Matrices gewährleistet sein. Um den universellen Einsatz zu belegen, wurden aus dem Archiv der Routinediagnostik entnommen und bereits in der "semi-nested"-PCR positiv getestete Proben zweimal auf dem LightCycler^{™} 480 geprüft. Kot-, Milch-, Sperma-, Blut-, Gewebe- und Umweltproben wurden ausgewählt (Figur 9).

MAP-DNA konnte in allen Matrices sicher in den beiden Läufen nachgewiesen werden. In Tabelle 5 sind die Cp-Werte der beiden Läufe sowie Mittelwert und Standardabweichung dargestellt.

**Tabelle 5: Ermittelte Cp-Werte der verschiedenen Matrices auf dem LightCycler^{™} 480 mit TaqMan Sonde.**

| | **Kot** | **Milch** | **Sperma** | **Blut** | **Gewebe** | **Umwelt** |
|---|---|---|---|---|---|---|
| **Lauf 1** | 25,07 | 33,53 | 27,83 | 30,00 | 33,26 | 29,33 |
| **Lauf 2** | 26,28 | 33,34 | 27,85 | 29,97 | 31,88 | 29,30 |
| **MW STBW** | 25,68 ± 0,86 | 33,44 ± 0,13 | 27, 84 ± 0,01 | 29,99 ± 0,02 | 32, 57 ± 0,98 | 29,32 ± 0,02 |

Die getesteten Kot-, Milch-, Sperma-, Blut-, Gewebe- und Umweltproben gaben ein eindeutiges fluoreszierendes Signal mit Cp-Werten zwischen 25,07 und 33,53. Das Ergebnis war reproduzierbar mit Standardabweichungen von 0,02 bis 0,98.

Für den Einsatz der Echtzeit-PCR zum Nachweis von Paratuberkulose bei Wiederkäuern können auch Kontrollsysteme für die Amplifikation (z.B. HEX-Kanal) bzw. Extraktion (z.B. Cy5-Kanal) eingesetzt werden, die über andere Label oder Marker unabhängig detektiert werden können. Dabei kann der Nachweis einer erfolgreichen Nukleinsäure-Extraktion z.B. über eine Amplifikation des Zielgens ß-Aktin erfolgen. Dem Fachmann sind weitere Kontrollsysteme für die Amplifikation bzw. Extraktion bekannt.

Das endgültige Protokoll der MAP TaqMan Echtzeit-PCR lautet wie folgt:

| | | |
|---|---|---|
| 95 °C | 10 min | |
| 95 °C | 15 sec | |
| 60 °C | 30 sec | 40 Zyklen |
| 72 °C | 35 sec | |

Die dem Archiv entnommenen Proben gaben auch bei der Analyse mit einem Kontrollsystem für die Amplifikation und Extraktion im FAM-Kanal (465-510 nm) ein reproduzierbares eindeutiges fluoreszierendes Signal mit durchschnittlichen Cp-Werten zwischen 23,58 und 33,29 sowie Standardabweichungen von 0,02 bis 0,98.

Der beschriebene Test wurde mit 13 Rindern, die als positiv auf MAP getestet waren, evaluiert. Dazu wurde DNA aus den Kotproben extrahiert und mit der beschriebenen Echtzeit-PCR auf MAP getestet. Hier zeigte sich, dass der Test MAP in Kotproben nachweisen konnte.

Schließlich wurden zur Bestätigung der Robustheit des Testes Organproben aus verschiedenen Darmbereichen zwei Kühe getestet. Wie aus den Tabellen unten zu erkennen, ist der erfindungsgemäße Test zum Nachweis der MAP geeignet.

**Tabelle 7: Quantitative Anwendung der IS900 TaqMan Echtzeit-PCR zum Nachweis von MAP in Gewebeproben von zwei an Paratuberkulose erkrankten Kühen. Darstellung der Cp-Werte dreimal gemessen im FAM-Kanal (465-510 nm).**

| **Tier** | **Probe** | **FAM-Kanal (465-510 nm)** | |
|---|---|---|---|
| **Tier A** | Duodenum | 32,68 ± 0,21 | |
| | Jejunum | 28,00 ± 0,12 | |
| | Ileum | 27,09 ± 0,08 | |
| | Caecum | 25,06 ± 0,04 | |
| | Ln caecalis | 33,00 ± 0,23 | |
| | | | |
| **Tier B** | Duodenum | 33,79 ± 0,69 | |
| | Jejunum | 34,33 ± 0,61 | |
| | Ileum | 25,52 ± 0,03 | |
| | Caecum | 24,53 ± 0,03 | |
| | Ln caecalis | 28,86 ± 0,11 | |

Alle Proben zeigten eine Amplifikationskurve im FAM-Kanal mit Cp-Werten von 24,53 bis 34,33 und Standardabweichungen von 0,03 bis 0,69.

Für eine quantitative Auswertung wurden, wie bereits oben beschrieben, DNA-Konzentration sowie MAP-Genomeinheiten pro Gramm Gewebe berechnet (Tabelle 8) und graphisch dargestellt (Figur 10).

**Tabelle 8: Quantitative Anwendung der IS900 TaqMan Echtzeit-PCR zum Nachweis von MAP in Gewebeproben zweier an Paratuberkulose erkrankter Kühen. Zur Quantifizierung von MAP in Gewebe wurden die DNA-Konzentration sowie die Zahl der Genomeinheiten pro Gramm Gewebe berechnet.**

| **Tier** | **Probe** | **DNA-Konzentration** | **MAP / g Gewebe** |
|---|---|---|---|
| **Tier A** | Duodenum | 7,99 × 10⁻¹⁴ | 6,21 × 10³ |
| | Jejunum | 4,04 × 10⁻¹² | 3,14 × 10⁵ |
| | Ileum | 8,13 × 10⁻¹² | 6,32 × 10⁵ |
| | Caecum | 3,57 × 10⁻¹¹ | 2,77 × 10⁶ |
| | Ln caecalis | 5,96 × 10⁻¹⁴ | 4,63 × 10³ |
| | | | |
| **Tier B** | Duodenum | 3,27 × 10⁻¹⁴ | 2,54 × 10³ |
| | Jejunum | 1,91 × 10⁻¹⁴ | 1,48 × 10³ |
| | Ileum | 2,57 × 10⁻¹¹ | 2,00 × 10⁶ |
| | Caecum | 5,17 × 10⁻¹¹ | 4,02 × 10⁶ |
| | Ln caecalis | 2,05 × 10⁻¹² | 1,59 × 10⁵ |

In einer Validierungsstudie wurden die kommerziell erhältlichen MAP-PCR-Kits Adiavet (Adiavet ParaTB Realtime), AB-TaqMan (Applied Biosystems Taq-ManMAP) und Vetmax (Applied Biosystems VetMax MAP Real Time PCR Screening Kit), sowie ein publizierter PCR-Assay in Bull et al. (Tim J. Bull et al., 2007, PloS One 2007(11), e1229) vergleichend zur hier beschriebenen Echtzeit-PCR getestet. Die Durchführung erfolgte bei den kommerziell erhältlichen Kits nach Herstellerangaben. Als Referenz wurde eine semi-nested-PCR (snPCR) eingesetzt (Münster P. et al, Vet Microbiol. 2011: 154(1-2): 197-201). Sie besitzt mit dem Nachweis von 1 Genomeinheit die höchste Sensitivität und ist allen "real-time"-Verfahren hinsichtlich der Sensitivität überlegen. Allerdings ist es allgemeiner Stand der Technik, dass solche snPCRs für Routineanwendungen wegen der extrem hohen Kontaminationsgefahr bei Massendurchsatz ungeeignet sind.

Die PCR-Läufe fanden auf einem Roche LightCycler 480 statt. Die snPCR lief klassisch auf einem konventionellen Thermocycler (Biometra "Trio").

Als Testproben standen 14 mittels semi-nested-PCR (sn-PCR) negativ getestete und 35 positiv getestete Kotproben aus der Routinedignostik zur Verfügung (N=49).

Die Ergebnisse der Untersuchung sind in Tabelle 9 für die positiv getesteten Kotproben zusammengefasst. Die Cp-Werte von zwei Untersuchungen wurden gemittelt. Standen die Ergebnisse nur von einer Untersuchung zur Verfügung, wurden diese für die weiteren Analysen verwendet. Die Validierung in Tabelle 9 stellt die Subtraktion der Cp-Werte dar. Zunächst wurde die TaqMan Echtzeit-PCR gegen alle kommerziell erhältlichen Vergleichs-PCRs geprüft. Hierzu wurde jeweils vom entsprechenden "TaqMan-PCR-Proben-CP-Wert" der korrespondierende "Vergleichs-PCR-Proben-CP-Wert" subtrahiert. Ein positiver Wert in der entsprechenden Spalte sagt aus, dass der TaqMan-PCR-Proben-CP-Wert höher war, als der subtrahierte Vergleichswert. Die PCR wäre demnach in der Sensitivität schlechter als die Vergleichs-PCR, da definitionsgemäß die Sensitivität umso höher ist je niedriger der CP-Wert ist. In gleicher Weise fand ein Vergleich zwischen der TaqMan-PCR und der publizierten PCR von Bull et al. statt, bei der das Endvolumen in der TaqMan Echtzeit-PCR auf die von Bull et al. beschriebenen PCR angepasst wurde (jeweils 50 µL statt 20 µL).

Die Vergleichsstudie kommt zu dem Ergebnis, dass die neu entwickelte Echtzeit-PCR allen Vergleichs-PCRs überlegen war. [Die TaqMan Echtzeit-PCR Plus schlug in der klassischen Variante (5 µl Template) alle Vergleichs-PCRs. Sie war auch ihrer eigenen Variante mit 8 µL Template knapp überlegen.]

**Tabelle 9:**

| Ergebnisse der Validierungsstudie | | | | | |
|---|---|---|---|---|---|
| **Lfd. NR.** | **snPCR** | **Taq-Man** | **TaqMan** | **Taq-Man** | **Taq-Man** |
| | | **Adiavet** | **ABTaqman** | **Vetmax** | **Bull et al.** |
| 1 | + | -7,55 | -2,92 | -2,06 | -5,31 |
| 2 | + | -7,44 | -2,91 | -2,00 | -5,59 |
| 3 | + | -2,73 | -1,54 | -1,02 | -4,62 |
| 4 | + | -2,67 | -1,23 | -0,79 | -4,30 |
| 5 | + | -6,95 | -2,42 | -1,23 | -4,57 |
| 6 | + | -3,65 | -1,61 | -0,47 | -3,37 |
| 7 | + | -10,07 | -1,39 | 0,74 | -2,32 |
| 8 | + | -6,49 | -2,31 | -1,37 | -4,44 |
| 9 | + | -7,95 | -8,10 | -10,97 | -4,89 |
| 10 | + | -2,19 | -1,82 | -6,31 | -0,27 |
| 11 | + | -4,85 | -10,82 | -13,86 | -8,96 |
| 12 | + | -2,56 | -7,86 | -13,82 | -7,72 |
| 13 | + | -9,21 | -9,43 | -12,34 | -3,61 |
| 14 | ++ | 0,41 | 2,75 | 1,73 | -2,59 |
| 15 | ++ | -3,85 | -0,28 | -1,40 | -4,75 |
| 16 | + | -11,20 | -11,20 | -11,20 | -4,39 |
| 17 | + | -11,95 | -11,95 | -11,95 | -4,36 |
| 18 | + | 3,59 | 9,85 | 8,76 | 5,59 |
| 19 | + | -10,11 | -10,11 | -10,11 | -2,92 |
| 20 | + | -12,24 | -12,24 | -12,24 | -5,77 |
| 21 | + | -12,34 | -13,03 | -13,03 | -3,17 |
| 22 | + | -6,48 | -6,48 | -6,48 | -6,48 |
| 23 | + | 0,00 | 8,07 | 6,93 | 4,29 |
| 24 | + | 2,94 | 2,81 | 0,00 | 0,73 |
| 25 | + | -8,91 | -9,15 | -11,62 | -9,78 |
| 26 | + | 0,00 | 0,00 | 0,00 | 5,98 |
| 27 | + | 3,05 | 2,88 | 0,00 | 5,21 |
| 28 | + | -12,30 | -12,30 | -12,30 | -4,65 |
| 29 | + | 0,00 | 0,00 | 0,00 | 1,63 |
| 30 | + | 0,00 | 0,00 | 0,00 | 1,91 |
| 31 | + | -13,52 | -13,52 | -13,52 | / |
| 32 | + | -11,59 | -11,59 | -11,59 | / |
| 33 | + | -10,40 | -10,40 | -10,40 | -10,40 |
| 34 | + | 0,00 | 0,00 | 0,00 | 0,00 |
| 35 | + | 0,00 | 0,00 | 0,00 | 0,00 |
| | | | | | |
| | **besser** | **25/35** | **25/35** | **24/35** | **24/33** |
| | **gleich** | **6/35** | **5/35** | **7/35** | **2/33** |
| | **schlech ter** | **4/35** | **5/35** | **4/35** | **7/33** |

Der obige Vergleich zeigt deutlich, dass das erfindungsgemäße Verfahren mit den erfindungsgemäßen Primern den im Stand der Technik beschriebenen Verfahren und den kommerziell erhältlichen Testkits in der Sensitivität überlegen ist.

### Kurze Beschreibung der Figuren

- **Figur 1:**: Graphische Darstellung der verwendeten Primer (MAP_{523-542f}/MAP₆₆₁₋₆₄₂ᵣ) sowie Patente der IS900.
- **Figur 2:**: Spezifitäts-Abklärung der Primer (MAP_{523-542f}/MAP₆₆₁₋₆₄₂ᵣ) mit nicht-Mykobakterienspezies. Dazu wurden amplifizierte PCR-Produkte mittels Gelektrophorese (1,5% Agarosegel) aufgetrennt.
- **Figur 3:**: Spezifitätsnachweis der TaqMan Echtzeit-PCR auf dem Light Cycler^{™} 480 anhand von Referenzstämmen (n = 14).
- **Figur 4:**: Spezifitäts-Abklärung der Primer (MAP_{523-542f}/MAP₆₆₁₋₆₄₂ᵣ) mit anderen Mykobakterien-Arten. Dazu wurden amplifizierte PCR-Produkte mittels Gelektrophorese (1,5% Agarosegel) aufgetrennt.
- **Figur 5:**: Sensitivitätsnachweis der Echtzeit-PCR anhand von ATCC 19698 MAP-DNA. **A:** 1 ng/µl, **B**: 100 pg/µl, **C:** 10 pg/µl, **D:** 1 pg/µl, **E:** 100 fg/µl, **F:** 10 fg/µl, G: 1 fg/µl, **NTC:** Steriles Wasser.
- **Figur 6:**: Reproduzierbarkeit der TaqMan Echtzeit-PCR anhand von 9 (3x3 Läufe) Wiederholungen (Effizienz = 1,97 ± 0,08; Fehler = 0,04 ± 0,02). Die Auswertung erfolgte mittels "2nd Derivative Maximum" Methode.
- **Figur 7:**: Reproduzierbarkeit der TaqMan Echtzeit-PCR anhand von 9 Wiederholungen (3x3 Läufe) (Effizienz = 2,07 ± 0,09; Fehler = 0,18 ± 0,03). Die Auswertung erfolgte mittels "Fit Point"-Methode.
- **Figur 8:**: Graphische Darstellung der Amplifikationskurven der Plasmid-DNA Verdünnungsreihen (1 ng/µl bis 1 fg/µl) hinterlegt mit H₂O **(A)** und Kot-DNA **(B)** auf dem LightCycler^{™} 480 mit TaqMan Sonde. Kurven v.l.n.r.: 1 ng/µl, 100 pg/µl, 10 pg/µl, 1 pg/µl, 100 fg/µl, 10 fg/µl, 1 fg/µl, Steriles Wasser (NTC).
- **Figur 9:**: Graphische Darstellung der Amplifikationskurven zum Nachweis von MAP-DNA aus verschiedenen Matrices auf dem LightCycler^{™} 480 mit TaqMan Sonde.
- **Figur 10:**: Quantitative Anwendung der IS900 TaqMan Echtzeit-PCR zum Nachweis von MAP in Gewebeproben. Gegenüberstellung von Tier A und Tier B. Die Balken stellen den Mittelwert sowie die Standardabweichung (3 Wiederholungen) der berechneten MAP-Konzentration in verschiedenen Geweben dar.

## Patentansprüche

1. Verfahren zum spezifischen Nachweis und ggf. zur Quantifizierung von *Mycobacterium avium ssp. paratuberculosis* (MAP) in einer Probe eines Individuums umfassend den Schritt:
- Nachweis der IS900 Region von einem MAP-Genom in einer Probe eines Individuums mit Hilfe einer Nukleinsäureamplifikation, wobei diese Amplifikation mit spezifischen Oligonukleotiden bestehend aus jeweils mindestens 15 aufeinanderfolgende Nukleotiden der Sequenzen gemäß Seq. ID Nr. 1 und Seq. ID Nr. 2 durchgeführt wird
wobei der Nachweis der IS900-Region mindestens eines MAP-Genoms das Vorhandensein von MAP im Individuum anzeigt.

2. Verfahren zum spezifischen Nachweis von MAP gemäß Anspruch 1, wobei der Nachweis mittels Polymerase-Ketten-Reaktion (PCR) erfolgt.

3. Verfahren zum spezifischen Nachweis von MAP gemäß Anspruch 1 oder 2, wobei der Nachweis mittels Echtzeit-PCR, bevorzugt als quantitative PCR, erfolgt.

4. Verfahren nach einem der vorherigen Ansprüche, **dadurch gekennzeichnet, dass** insbesondere bei der PCR, der Nachweis der IS900-Region die Verwendung einer Nukleinsäuresonde umfasst, die an die IS900-Region des MAP-Genoms und/oder an einer Nukleinsäure komplementär zu der IS900-Region des MAP-Genoms, hybridisiert.

5. Verfahren nach einem der Ansprüche 1 bis 4, wobei der Nachweis mittels quantitativer Echtzeit-PCR unter Verwendung einer Sonde aus einem Oligonukleotid mit mindestens 15 aufeinanderfolgende Nukleotiden gemäß Seq. ID Nr. 3 erfolgt.

6. Verfahren nach einem der vorherigen Ansprüche, **dadurch gekennzeichnet, dass** die Probe eine aus Kot-, Milch-, Blut-, Sperma-, Gewebe-, Organ- und Umweltproben ist.

7. Verfahren nach einem der vorherigen Ansprüche, **dadurch gekennzeichnet, dass** die Probe von Wiederkäuern, insbesondere Rinder, Schafe und Ziegen oder von Menschen stammt.

8. Verfahren nach einem der vorherigen Ansprüche, wobei der spezifische Nachweis von MAP mittels Echtzeit-PCR und Verwendung der Oligonukleotide gemäß Seq ID No. 1, Seq ID No. 2 und Seq ID No. 3 erfolgt.

9. Test-Kit zum spezifischen Nachweis von MAP in einer Probe mittels Amplikationsverfahren, insbesondere mittels PCR, **dadurch gekennzeichnet, dass** das Test-Kit Oligonukleotide zur Amplifizierung von der MAP IS900-Region mit jeweils mindestens 15 aufeinanderfolgende Nukleotiden gemäß Seq. ID Nr. 1 und 2 umfasst.

10. Test-Kit gemäß Anspruch 9, **dadurch gekennzeichnet, dass** es weiterhin eine Sonde mit mindestens 15 aufeinanderfolgende Nukleotiden gemäß Seq. ID Nr. 3 umfasst.

11. Test-Kit gemäß Anspruch 9 oder 10 **dadurch gekennzeichnet, dass** es weiterhin Mittel zur Gewinnung der genomischen DNA aus Proben, insbesondere Kot-, Milch-, Blut-, Sperma-, Organ-, Gewebe- und Umweltproben aufweist.

12. Test-Kit nach einem der Ansprüche 9 bis 11, **dadurch gekennzeichnet, dass** es weiterhin Mittel zur Durchführung einer PCR, insbesondere Echtzeit-PCR aufweist.

13. Verwendung eines Test-Kits gemäß einem der Ansprüche 9 bis 12 zum spezifischen Nachweis von IS900 von MAP in Proben, insbesondere Kot-, Milch-, Blut-, Sperma-, Gewebe-, Organ- und Umweltproben in einem Individuum.

14. Oligonukleotide zum spezifischen Nachweis und, gegebenenfalls, zur Quantifizierung von MAP in einer Probe eines Individuums, **dadurch gekennzeichnet, dass** die Oligonukleotide folgende Nukleotidsequenzen aufweisen: ein Oligonukleotid mit mindestens 15 aufeinanderfolgenden Nukleotiden der Seq. ID Nr. 1, ein Oligonukleotid mit mindestens 15 aufeinanderfolgenden Nukleotiden der Seq. ID Nr. 2, und, gegebenenfalls, ein Oligonukleotid mit mindestens 15 aufeinanderfolgenden Nukleotiden der Seq. ID Nr. 3, bevorzugt die Oligonucleotide gemäß Seq ID No. 1, Seq. ID No. 2 und Seq. ID No. 3.

15. Verwendung der Oligonukleotide gemäß Anspruch 14 zum spezifischen Nachweis und, gegebenenfalls, zur Quantifizierung, von *Mycobacterium avium* ssp. *paratuberculosis* (MAP) insbesondere mittels quantitativer Echtzeit-PCR.

## Claims

1. A method for the specific detection and optionally quantification of *Mycobacterium avium ssp. Paratuberculosis* (MAP) in a sample of an individual comprising the step of:
- detecting the IS900 region of a MAP-genome in a sample from an individual by means of a nucleic acid amplification, whereby this amplification is effected with specific oligonucleotides respectively consisting of at least 15 consecutive nucleotides of the sequences according to SEQ ID No. 1 and SEQ ID No. 2,
wherein the detection of the IS900 region of at least one MAP-genome indicates the presence of MAP in the individual.

2. The method for the specific detection of MAP according to claim 1, whereby the detection is effected by polymerase chain reaction (PCR).

3. The method for the specific detection of MAP according to claim 1 or 2, whereby the detection is effected by real time PCR, preferably quantitative PCR.

4. The method according to any one of the preceding claims **characterized in that** the detection in particular when with the PCR, of the IS900 region comprises the use of a nucleic acid probe which hybridizes with the IS900 region of the MAP-genome and/or with a nucleic acid complementary to the IS900 region of the MAP-genome.

5. The method according to any one of claims 1 to 4 whereby the detection is effected by quantitative real time PCR with a use of a probe of an oligonucleotide with at least 15 consecutive nucleotides according to SEQ ID No. 3.

6. The method according to any one of the preceding claims, **characterized in that** the sample is one of fecal, milk, blood, semen, tissue, organ and environmental samples.

7. The method according to any one of the preceding claims, **characterized in that** the sample derives from ruminants, in particular, cattle, sheep and goats, or from man.

8. The method according to any one of the preceding claims, whereby the specific detection of MAP is effected by real time PCR and use of the oligonucleotides according to SEQ ID No. 1, SEQ ID No. 2 and SEQ ID No. 3.

9. Test-kitfor specific detection of MAP in a sample by amplification methods, in particular by PCR, **characterized in that** the test kit comprises oligonucleotides for amplification of the MAP IS900 region with at least 15 consecutive nucleotides according to SEQ ID No. 1 and 2, respectively.

10. The test-kit according to claim 9, **characterized in that** it further comprises a probe with at least 15 consecutive nucleotides according to SEQ ID No. 3.

11. The test-kit according to claim 9 or 10, **characterized in that** it further contains means for obtaining the genomic DNA from samples, in particular, fecal, milk, blood, semen, organ, tissue and environmental samples.

12. The test-kit according to any one of claims 9 to 11, **characterized in that** it further contains means for performing a PCR, in particular, real time PCR.

13. Use of a test-kit according to any one of claims 9 to 12 for specific detection of IS900 from MAP in samples, in particular, fecal, milk, blood, semen, tissue, organ and environmental samples in an individual.

14. Oligonucleotides for specific detection and, optionally, for quantification of MAP in a sample from an individual, **characterized in that** the oligonucleotides have the following nucleic acid sequences: an oligonucleotide having at least 15 consecutive nucleotides of SEQ ID No. 1, an oligonucleotide having at least 15 consecutive nucleotides of SEQ ID No. 2 and, optionally, an oligonucleotide having at least 15 consecutive nucleotides of SEQ ID No. 3, in particular, the oligonucleotides of SEQ ID No. 1 , SEQ ID No. 2 and SEQ ID No. 3.

15. Use of oligonucleotides according to claim 14 for specific detection and, optionally, quantification of *Mycobacterium avium ssp. paratuberculosis* (MAP), particularly by quantitative real time PCR.

## Revendications

1. Procédé destiné à la détermination spécifique et, le cas échéant, à la quantification de Mycobacterium avium ssp. paratuberculosis (MAP) dans un échantillon prélevé chez un individu, lequel procédé comprend la phase :
- la détermination de la région IS900 d'un génome de MAP dans un échantillon prélevé chez un individu à l'aide d'une amplification de l'acide nucléique, selon lequel cette amplification est réalisée avec des oligonucléotides spécifiques constitués de respectivement au moins 15 nucléotides consécutifs des séquences conformément à la séq. ID n° 1 et à la séq. ID n° 2 ;
selon lequel la détermination de la région IS900 d'au moins un génome de MAP révèle la présence de MAP chez un individu.

2. Procédé destiné à la détermination spécifique de MAP selon la revendication 1, selon lequel la détermination proprement dite est réalisée au moyen de la réaction en chaîne de la polymérase (procédé PCR).

3. Procédé destiné à la détermination spécifique de MAP selon la revendication 1 ou 2, selon lequel la détermination proprement dite est réalisée au moyen d'un procédé PCR en temps réel, de préférence sous la forme d'un procédé PCR quantitatif.

4. Procédé selon l'une des revendications précédentes, **caractérisé en ce que**, en particulier lors de la mise en oeuvre du procédé PCR, la détermination de la région IS900 comprend le recours à une sonde d'acide nucléique, laquelle permet l'hybridation à la région IS900 du génome de MAP et/ou à un acide nucléique de façon complémentaire à la région IS900 du génome de MAP.

5. Procédé selon l'une des revendications 1 à 4, selon lequel la détermination est réalisée au moyen du procédé PCR quantitatif en temps réel en recourant à une sonde issue d'un oligonucléotide comprenant au moins 15 nucléotides consécutifs conformément à la séq. ID n° 3.

6. Procédé selon l'une des revendications précédentes, **caractérisé en ce que** l'échantillon en question est issu d'échantillons de matières fécales, d'échantillons de lait, d'échantillons de sang, d'échantillons de sperme, d'échantillons de tissu, d'échantillons d'organes et d'échantillons de l'environnement.

7. Procédé selon l'une des revendications précédentes, **caractérisé en ce que** l'échantillon provient de ruminants, en particulier de bovins, de moutons et de caprins, ou d'êtres humains.

8. Procédé selon l'une des revendications précédentes, selon lequel la détermination spécifique de MAP est réalisée au moyen du procédé PCR en temps réel et en recourant à l'oligonucléotide conformément à la séq. ID n° 1, la séq. ID n° 2 et la séq. ID n° 3.

9. Kit de test destiné à la détermination spécifique de MAP dans un échantillon au moyen d'un procédé d'amplification, en particulier au moyen du procédé PCR, **caractérisé en ce que** le kit de test comprend des oligonucléotides en vue de l'amplification de la région IS900 d'un génome de MAP avec respectivement au moins 15 nucléotides consécutifs conformément à la séq. ID n° 1 et séq. ID n° 2.

10. Kit de test selon la revendication 9, **caractérisé en ce que** le kit de test comprend en outre une sonde comprenant au moins 15 nucléotides consécutifs conformément à la séq. ID n° 3.

11. Kit de test selon la revendication 9 ou 10 **caractérisé en ce que** le kit de test présente en outre des moyens destinés à la récupération de l'ADN génomique prélevé dans des échantillons, en particulier dans des échantillons de matières fécales, dans des échantillons de lait, dans des échantillons de sang, dans des échantillons de sperme, dans des échantillons d'organes, dans des échantillons de tissu et dans des échantillons de l'environnement.

12. Kit de test selon l'une des revendications 9 à 11, **caractérisé en ce que** le kit de test présente en outre des moyens destinés à la réalisation d'un procédé PCR, en particulier d'un procédé PCR en temps réel.

13. Utilisation d'un kit de test selon l'une des revendications 9 à 12 en vue de la détermination spécifique de la région IS900 d'un génome de MAP dans des échantillons, en particulier dans des échantillons de matières fécales, dans des échantillons de lait, dans des échantillons de sang, dans des échantillons de sperme, dans des échantillons d'organes, dans des échantillons de tissu et dans des échantillons de l'environnement, lesquels sont prélevés chez un individu.

14. Oligonucléotide destiné à la détermination spécifique et, le cas échéant, à la quantification de MAP dans un échantillon prélevé chez un individu, **caractérisé en ce que** l'oligonucléotide en question présente les séquences nucléotidiques suivantes : un oligonucléotide comprenant au moins 15 nucléotides consécutifs de la séq. ID n° 1, un oligonucléotide comprenant au moins 15 nucléotides consécutifs de la séq. ID n° 2 et, le cas échéant, un oligonucléotide comprenant au moins 15 nucléotides consécutifs de la séq. ID n° 3, de préférence les oligonucléotides conformément à la séq. ID n° 1, la séq. ID n° 2 et la séq. ID n° 3.

15. Utilisation des oligonucléotides selon la revendication 14 destinée à la détermination spécifique et, le cas échéant, à la quantification de Mycobacterium avium Ssp. paratuberculosis (MAP), en particulier au moyen d'un procédé PCR quantitatif en temps réel.
